(19) Europäisches Patentamt
European Patent Office
Office européen des brevets

(11) **EP 3 125 161 A1**

(12) **EUROPEAN PATENT APPLICATION**
published in accordance with Art. 153(4) EPC

(43) Date of publication:
**01.02.2017 Bulletin 2017/05**

(21) Application number: **14887478.7**

(22) Date of filing: **25.03.2014**

(51) Int Cl.:
**G06N 5/04** (2006.01)    **G06Q 50/22** (2012.01)

(86) International application number:
**PCT/JP2014/058166**

(87) International publication number:
**WO 2015/145555 (01.10.2015 Gazette 2015/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Hitachi, Ltd.**
**Chiyoda-ku**
**Tokyo 100-8280 (JP)**

(72) Inventors:
• **HIROKI Keiichi**
 **Tokyo 100-8280 (JP)**
• **MIYOSHI Toshinori**
 **Tokyo 100-8280 (JP)**

(74) Representative: **Addiss, John William et al**
 **Mewburn Ellis LLP**
 **City Tower**
 **40 Basinghall Street**
 **London EC2V 5DE (GB)**

(54) **PROBABILISTIC INFERENCE SYSTEM**

(57)    A probabilistic inference system is provided with: a pre-modification model input unit that receives the input of a probabilistic inference model; a model modification execution unit that outputs a modified probabilistic inference model by modifying the probabilistic inference model; an inference calculation cost estimation unit that calculates a calculation cost when a probabilistic inference process is performed using the modified probabilistic inference model; an inference error estimation unit that estimates the magnitude of inference error that could be caused in a certain designated random variable in the probabilistic inference model when the probabilistic inference process is performed using the modified probabilistic inference model, compared with when the probabilistic inference process is performed using the probabilistic inference model; an adopted model selection unit that selects the probabilistic inference model to be adopted based on a probabilistic inference condition regarding the calculation cost and the inference error; and a modified model output unit that outputs the adopted probabilistic inference model.

FIG. 1

Disease onset prediction device

Input unit — 108

Output unit — 109

Computing device — 110

Memory — 111

Storage medium — 107

Disease state transition model input unit — 101

Disease state transition model modification unit — 102

Probabilistic inference condition input unit — 103

Analysis subject medical data input unit — 104

Probabilistic inference execution unit — 105

Prediction result output unit — 106

EP 3 125 161 A1

**Description**

Technical Field

The present invention relates to a probabilistic inference system which uses a probabilistic inference model.

Background Art

Methods are widely known that estimate an unknown event or a future event by performing probabilistic inference using a probabilistic inference model, such as a Bayesian network which is a probabilistic model of the causal relationships of past data. In the probabilistic inference using a Bayesian network, it is known that the amount of calculation necessary for probabilistic inference increases as the Bayesian network becomes more complex, and that it may become impossible to perform exact probabilistic inference in a realistic time. Accordingly, a probabilistic inference technique called approximate inference may be used, which is capable of performing inference with a small amount of calculation at the expense of a decrease in inference accuracy. An example of an approximate inferencing technique reduces the amount of calculation by modifying a Bayesian network itself, as disclosed in Patent Literature 1.

Citation List

Patent Literature

Patent Literature 1: U.S. Patent No. 8447710

Summary of Invention

Technical Problem

The relative merits of the conventional approximate inference techniques, such as the technique discussed in Patent Literature 1, have often been evaluated in terms of the amount of calculation and accuracy (the smallness of error from an exact inference result) of probabilistic inference. Accuracy evaluation often involves consideration of an average value or a maximum value of estimation errors with respect to all events, and the conventional approximate inference techniques have also placed emphasis on minimizing such values. As a consequence, there has been the tendency of an error occurring in a certain value width regardless of the importance of the event.

In addition, the conventional approximate inference techniques have the tendency to cause the same degree of errors with respect to an event with a low occurrence probability and with respect to an event with a high occurrence probability. Just as the seriousness differs between an error of 1% with respect to an event with an occurrence probability of 20% and an error of 1% with respect to an event with an occurrence probability of 1%, the tolerance with respect to the magnitude of the error varies depending on the original occurrence probability.

Due to the above-described circumstance, there has been the problem of reduced estimation accuracy when estimating the occurrence of an event of which the inherent occurrence probability is low but which is important, such as an accident, a failure, or the onset of serious disease, by approximate inference. In addition, there is often a trade-off between the accuracy of probabilistic inference and the amount of calculation for probabilistic inference, and there has been the problem of difficulty, when adjusting their balance, in making adjustment for the accuracy of probabilistic inference of a specific event rather than for the accuracy of probabilistic inference of all events.

An object of the present invention is to provide a probabilistic inference system which can probabilistically infer the accuracy of a designated specific event with high accuracy and at high speed, and which can make adjustment focusing on the inference accuracy of a specific event when adjusting the balance between the accuracy of probabilistic inference and the amount of calculation.

Solution to Problem

In order to solve the problem, the configurations set forth in the claims are adopted, for example. The present application includes a plurality of means for solving the problem. For example, there is provided a probabilistic inference system including a pre-modification model input unit that receives an input of a probabilistic inference model; a model modification execution unit that outputs a modified probabilistic inference model by modifying the probabilistic inference model; an inference calculation cost estimation unit that calculates a calculation cost when a probabilistic inference process is performed using the modified probabilistic inference model; an inference error estimation unit that estimates a magnitude of inference error that could be caused in a certain designated random variable in the probabilistic inference model when the probabilistic inference process is performed using the modified probabilistic inference model, compared with when the probabilistic inference process is performed using the probabilistic inference model; an adopted model selection unit that selects a probabilistic inference model to be adopted based on a probabilistic inference condition regarding the calculation cost and the inference error; and a post-modification model output unit that outputs the adopted probabilistic inference model.

Advantageous Effects of Invention

According to the present invention, by modifying a probabilistic inference model, a designated specific event can be probabilistically inferred at high speed and with high accuracy, and adjustment focusing on the inference accuracy of a specific event can be made when adjusting the balance between the accuracy of probabilistic inference and the amount of calculation.

Additional features of the present invention will become apparent from the following descriptions and the attached drawings. Problems, configurations, and effects other than those mentioned above will become apparent from the following description of embodiments.

Brief Description of Drawings

FIG. 1 is a configuration diagram of a disease onset prediction device according to a first embodiment.
FIG. 2 is a configuration diagram of a disease state transition model modification unit according to the first embodiment.
FIG. 3 is a flowchart describing a process by the disease onset prediction device according to the first embodiment.
FIG. 4 is a flowchart describing a process by the disease state transition model modification unit according to the first embodiment.
FIG. 5 is an example of a disease state transition model according to the first embodiment.
FIG. 6 is an example of a modified disease state transition model according to the first embodiment.
FIG. 7 illustrates cliques.
FIG. 8 describes a process by an inference error estimation unit according to the first embodiment.
FIG. 9 is an example of comparisons of a plurality of modified disease state transition models in terms of the amount of calculation and estimation error in the first embodiment.
FIG. 10 describes a process by an adopted model selection unit according to the first embodiment.
FIG. 11 is an example of an interface of a probabilistic inference condition input unit according to the first embodiment.
FIG. 12 describes a process by a disease state transition model modification unit according to a second embodiment.

Description of Embodiments

In the following, embodiments of the present invention will be described with reference to the attached drawings. While the attached drawings illustrate specific embodiments in accordance with the principle of the present invention, these are for the purpose of facilitating an understanding of the present invention and not to be taken in a limited sense.

First embodiment

In the present embodiment, an example of a disease onset prediction device will be described which predicts the future disease occurrence probability of a subject of analysis on the basis of medical data, such as medical examination results, medical interview results, clinical history, and medical records.

The medical data refer to data including personal medical and health information, such as the medical record and test values of individual subjects. For example, the medical data include test values measured at the time of a health checkup or a medical interview, such as height, body weight, BMI, blood pressure, cholesterol, and blood sugar level. Other examples of medical data include lifestyle habits information, such as the presence or absence of smoking; the presence or absence of daily perspiring exercise; the presence or absence of drinking; and the sleep state. Other examples of medical data include clinical history information, such as the history of disease names diagnosed at a medical institution. Yet other examples of medical data may include medical record information, such as the prescribed pharmaceutical products, performed medical acts, and medical expenses.

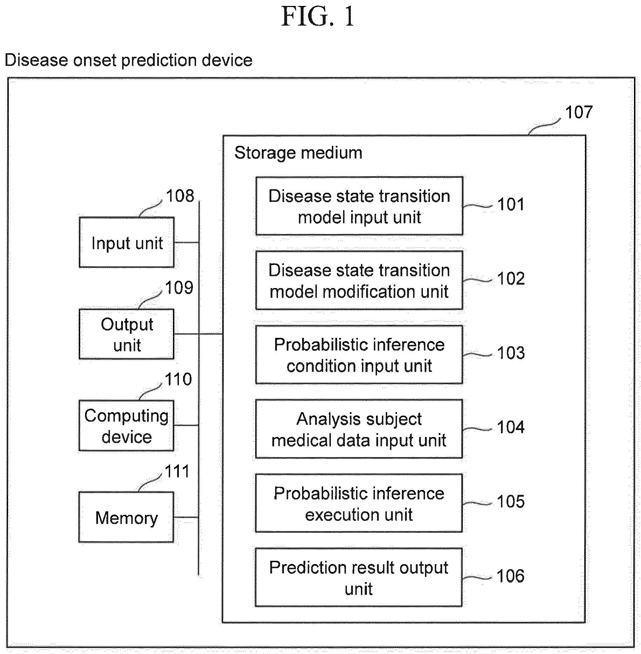

FIG. 1 is a configuration diagram of a disease onset prediction device according to the present embodiment. The disease onset prediction device is provided with an input unit 108; an output unit 109; a computing device 110; a memory 111; and a storage medium 107. The input unit 108 is a human interface, such as a mouse and keyboard, which is used to accept an input to the disease onset prediction device. The output unit 109 is a display, a printer or the like that outputs the result of computation by the disease onset prediction device. The storage medium 107 is a storage device that stores various programs for implementing analysis processes by the disease onset prediction device, results of execution of processes, and the like. In the storage medium 107, there are stored various programs for a disease state transition model input unit 101, a disease state transition model modification unit 102, a probabilistic inference condition input unit 103, a analysis subject medical data input unit 104, a probabilistic inference execution unit 105, and a prediction result output unit 106.

In the memory 111, the various programs stored in the storage medium 107 are loaded. The computing device 110 is a computing device (processor) that executes the programs loaded in the memory 111, and may include a CPU or a GPU, for example. The processes and computations described below are executed by the computing device 110.

The disease state transition model input unit 101 accepts the input of a disease state transition model. The disease state transition model refers to a probabilistic model describing the statistical probabilistic causal relationships of items of medical data, such as medical examination results, medical interview results, clinical history, and medical records. In the present embodiment, the disease state transition model is implemented in the form of a Bayesian network which is statistically constructed from past medical data that have been accumulated in large volumes. In the Bayesian network, when some variables are observed, the probability distribution of other variables can be determined. The computation based on the probability calculation performed at this time is referred to as probabilistic inference. The model that can be applied for the present invention is not limited to the Bayesian network, and may be implemented in the form of other graphical models that describe causal relationships by probability.

The disease state transition model modification unit 102 modifies the input disease state transition model so as to decrease the calculation cost required at the time of execution of probabilistic inference calculation on the disease state transition model. The configuration of the disease state transition model modification unit will be described later.

The probabilistic inference condition input unit 103 accepts the input of probabilistic inference conditions when probabilistic inference is performed using the disease state transition model. The probabilistic inference conditions refer to the conditions to be satisfied when executing probabilistic inference, and include the required accuracy for each estimation item and/or the permissible amount of time required for execution of the probabilistic inference calculation. For example, the conditions require that the estimation error of the occurrence probability of diabetes be not more than 5%, or that the probabilistic inference execution time be not longer than 1 second per case. In the present embodiment, the probabilistic inference condition input unit 103 is implemented in the form of a program for causing an interface to be displayed on a display screen of the output unit 109 and for accepting the input from the input unit 108.

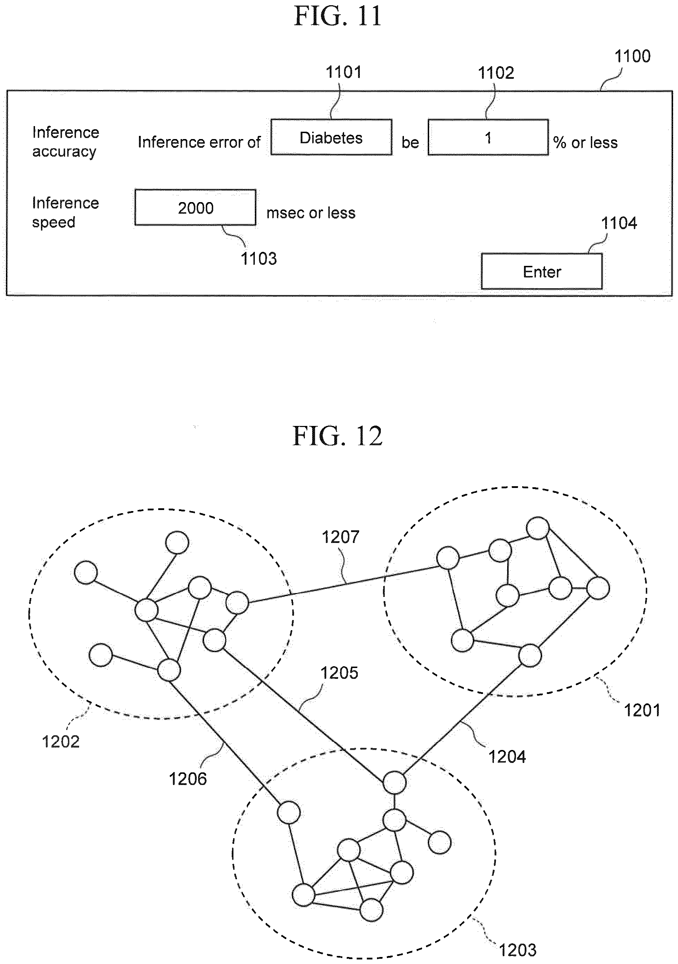

FIG. 11 is an example of an interface caused to be displayed on the display screen by the probabilistic inference condition input unit 103. The interface 1100 on the screen is provided with entry boxes 1101, 1102, and 1103 for the probabilistic inference execution conditions. In the first entry box 1101, the item for which inference accuracy is to be designated is entered. In the illustrated example, the item "diabetes" is entered. In the second entry box 1102, a lower limit of inference accuracy is entered. In the third entry box 1103, an upper limit of inference execution time is entered. After entries are made in the entry boxes 1101, 1102, and 1103, a button 1104 is depressed, whereby the probabilistic inference condition input unit 103 determines the probabilistic inference conditions.

The analysis subject medical data input unit 104 accepts the input of medical data concerning the subject of analysis, such as medical examination results, medical interview results, clinical history, and medical records.

The probabilistic inference execution unit 105, using the disease state transition model modified by the disease state transition model modification unit 102, and on the basis of the medical data accepted by the analysis subject medical data input unit 104, performs probabilistic inference calculation for estimating the disease onset probability for the subject of analysis. Examples of probabilistic inference calculation techniques on the Bayesian network include a technique combining a junction tree algorithm and a message-passing algorithm, and a bucket elimination algorithm. The probabilistic inference execution unit 105 according to the present embodiment is supposed to be a computer in which program software implementing probabilistic inference calculations combining the junction tree algorithm and the message-passing algorithm is mounted. Probabilistic inference calculations not based on the above-described algorithms are also included in the scope of application of the present invention.

The prediction result output unit 106 outputs to the output unit 109 the disease onset probability for the subject of analysis that has been output from the probabilistic inference execution unit 105.

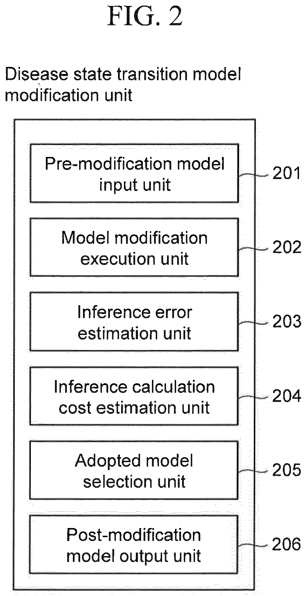

FIG. 2 is an example of a configuration diagram of the disease state transition model modification unit 102. The disease state transition model modification unit 102 is provided with a pre-modification model input unit 201; a model modification execution unit 202; an inference error estimation unit 203; an inference calculation cost estimation unit 204; an adopted model selection unit 205; and a post-modification model output unit 206.

The pre-modification model input unit 201 accepts a disease state transition model prior to modification. The model modification execution unit 202 modifies the disease state transition model accepted by the pre-modification model input unit 201, and creates a plurality of disease state transition models. The inference error estimation unit 203, with respect to each of the plurality of disease state transition models, calculates an estimated inference error. The inference calculation cost estimation unit 204 calculates an inference calculation cost for each of the plurality of disease state transition models. The adopted model selection unit 205 determines the disease state transition model to be adopted, based on the estimated inference error and inference calculation cost that have been calculated. Specifically, the adopted model selection unit 205 determines the disease state transition model to be adopted by determining whether the probabilistic inference conditions accepted by the probabilistic inference condition input unit 103 are satisfied. The adopted disease state transition model is output by the post-modification model output unit 206.

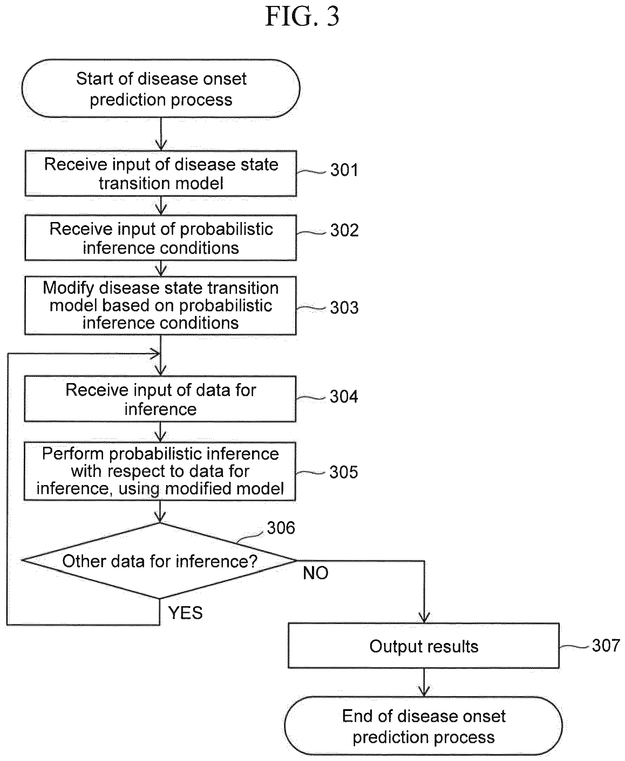

The operation of the disease onset prediction device will be described. FIG. 3 is a flowchart describing the operation of the disease onset prediction device. In step 301, the disease state transition model input unit 101 receives the input of a disease state transition model. In step 302, the probabilistic inference condition input unit 103 receives the input of probabilistic inference conditions via the interface displayed on the screen.

In step 303, the disease state transition model modification unit 102 creates a plurality of disease state transition models by modifying the disease state transition model, and determines from the plurality of disease state transition models the disease state transition model to be used for probabilistic inference, on the basis of the probabilistic inference conditions. Then, in step 304, the analysis subject medical data input unit 104 receives the input of medical data to be analyzed.

In step 305, the probabilistic inference execution unit 105 performs probabilistic inference with respect to the received medical data, using the adopted disease state transition model, and calculates the incidence rate of a disease. In step 306, it is determined whether there is other input data (medical data) to be analyzed. If there is other such data, the process returns to step 304 and is continued for the new medical data. If there is no other medical data to be analyzed in step 306, the process proceeds to step 307. In step 307, the prediction result output unit 106 outputs the result of probabilistic inference to the output unit 109, and the process ends.

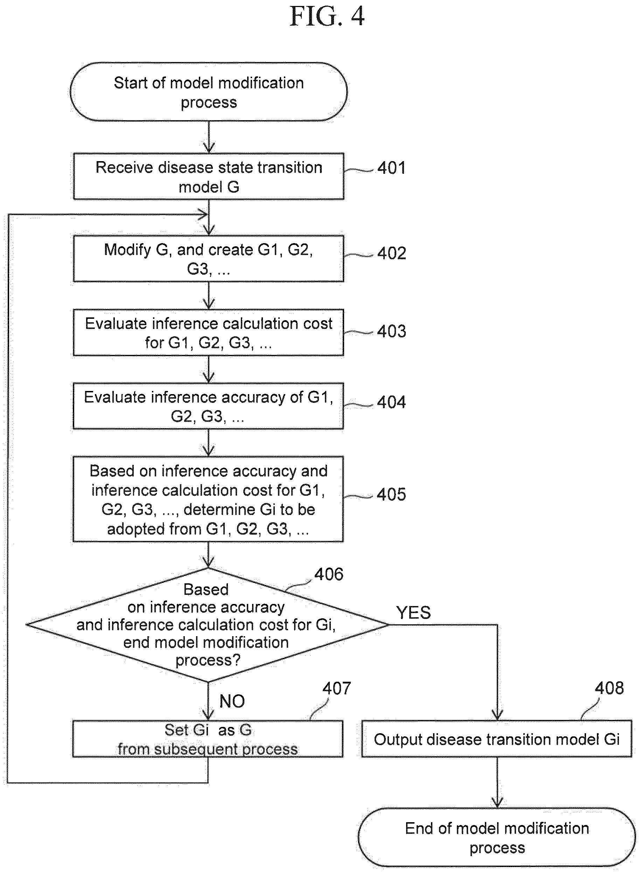

The operation of the disease state transition model modification unit 102 will be described. FIG. 4 is a flowchart describing the operation of the disease state transition model modification unit 102. In step 401, the pre-modification model input unit 201 receives the input of a pre-modification disease state transition model G received by the disease state transition model input unit 101. In step 402, the model modification execution unit 202 modifies the disease state transition model G by a plurality of methods, and creates modified disease state transition models G1, G2, G3, ..., and Gn.

In step 403, the inference calculation cost estimation unit 204 calculates the inference calculation cost for each of the modified models G1, G2, G3, ..., and Gn. In step 404, the inference error estimation unit 203 calculates the estimated inference accuracy of each of the disease state transition models G1, G2, G3, ..., and Gn.

In step 405, the adopted model selection unit 205, on the basis of the estimated inference error and inference calculation cost for each of the disease state transition models G1, G2, G3, ..., and Gn, determines a disease state transition model Gi to be adopted. In step 406, the adopted model selection unit 205 determines whether the disease state transition model Gi already satisfies the probabilistic inference conditions entered in the probabilistic inference condition input unit 103, or if there is the possibility of satisfying by continuing the process, and determines whether to end the model modification process or not. If the probabilistic inference conditions are already satisfied, or if there is no possibility of the probabilistic inference conditions being satisfied by continuing the process, the process proceeds to step 408. In step 408, if the probabilistic inference conditions are already satisfied, the post-modification model output unit 206 outputs the modified model Gi, and the process ends. If there is no possibility of being satisfied by continuing the process, the modified model Gi may be output as is, or the modified model that has been adopted as Gi in the previous process may be output. If there is no possibility of being satisfied by continuing the process, the process may be branched to another process, such as resetting the probability estimate conditions without outputting Gi.

In step 406, if the probabilistic inference conditions are not satisfied but there is the possibility of being satisfied by continuing the process, the adopted model selection unit 205 determines that the model modification process continue, and proceeds to step 407. In step 407, the modified model Gi is set as G. Thereafter, the process returns to step 402, and continues the model modification process.

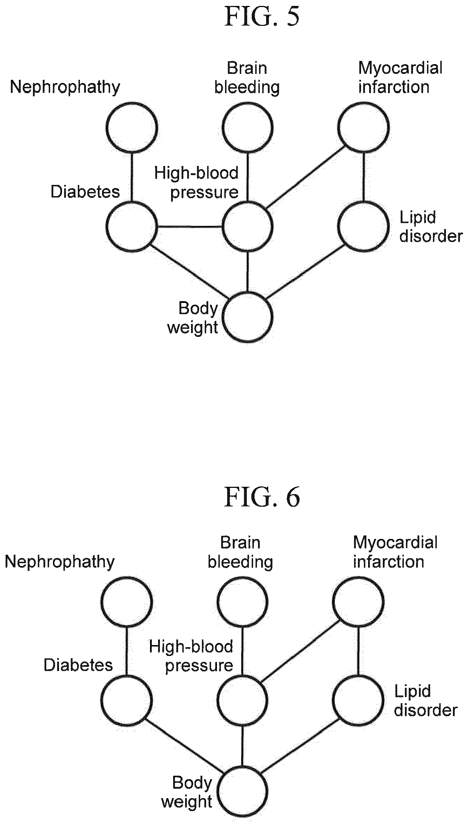

An example of the process in step 402 of modifying the disease state transition model will be described. The disease state transition model is modified by deleting one of links in the Bayesian network. The links represent the probabilistic dependencies between random variables. FIG. 5 is an example of the disease state transition model received by the pre-modification model input unit 201. The disease state transition model includes random variables and links representing the probabilistic dependencies between the random variables. FIG. 6 is an example of a Bayesian network obtained by deleting one link from the disease state transition model of FIG. 5. By deleting the one link, the Bayesian network of FIG. 5 is modified to the Bayesian network of FIG. 6. Here, the link between diabetes and high-blood pressure is deleted. Generally, when a link in a Bayesian network is deleted, the calculation cost for probabilistic inference is decreased; however, inference accuracy is also decreased. The calculation cost and inference accuracy that are decreased vary depending on which link is deleted. In the present embodiment, the plurality of disease state transition models G1, G2, G3, ..., and Gn are created for when each of all links in the graph of the disease state transition model is deleted. The method for creating the plurality of disease state transition models is not limited to the illustrated example, and other methods may be employed. For example, a plurality of disease state transition models may be created by performing deletion with respect to any desired links in the graph of the disease state transition model.

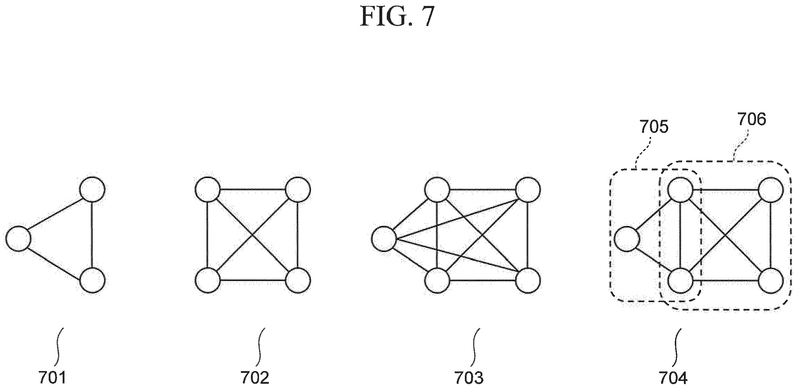

An example of the process in step 403 of calculating the inference calculation cost of the modified disease state transition model will be described. When the junction tree algorithm and the message-passing algorithm are used, the calculation cost of probabilistic inference by Bayesian network is determined by the state of a group of state variables called clique. A clique is a set of state variables, and all of the state variables included in a clique are required to be mutually connected by links. FIG. 7 shows examples of cliques. Clique 701 includes three state variables. Clique 702 includes four state variables. Clique 703 includes five state variables. Meanwhile, the configuration designated by 704 includes nodes that are not connected with links, so that the configuration does not constitute one clique as a whole but comprises individual cliques 705 and 706. The calculation cost of probabilistic inference by Bayesian network when the junction tree algorithm and the message-passing algorithm are used can be determined by the mathematical expression 1.

[Mathematical Expression 1]

$$\sum_{Message} s\_state * (r\_node + 2*s\_node) + r\_state * (r\_node + 2*b\_node) + \sum_{Clique} c\_state * c\_neighbor^2$$

where s_state is the product of state numbers of random variables included in a message transmission-side clique; r_state is the product of state numbers of random variables included in a message reception-side clique; s_node is the number of random variables included in the transmission-side clique; r_node is the number of random variables included in the reception-side clique; b_node is the number of random variables commonly included in the transmission-side clique and the reception-side clique; and c_state is the state number of a clique. The state number of a clique is the product of all state numbers of random variables included in the clique. C_neighbor is the number of neighboring cliques to a clique; namely the number of links a clique has.

An example of the process in step 404 of estimating the inference error of the modified disease state transition model will be described with reference to FIG. 5, FIG. 6, and FIG. 8. The inference error estimation unit 203 estimates the magnitude of the inference error that could be caused in certain designated random variables in the probabilistic inference model when a probabilistic inference process is performed using a modified probabilistic inference model, compared with when the probabilistic inference process is performed using a pre-modification probabilistic inference model. In the following, an example will be considered in which the inference error in the inference result for the incidence rate of myocardial infarction when the probabilistic inference process is performed using the Bayesian network of FIG. 6 is determined, compared with when the probabilistic inference process is performed using the Bayesian network of FIG. 5.

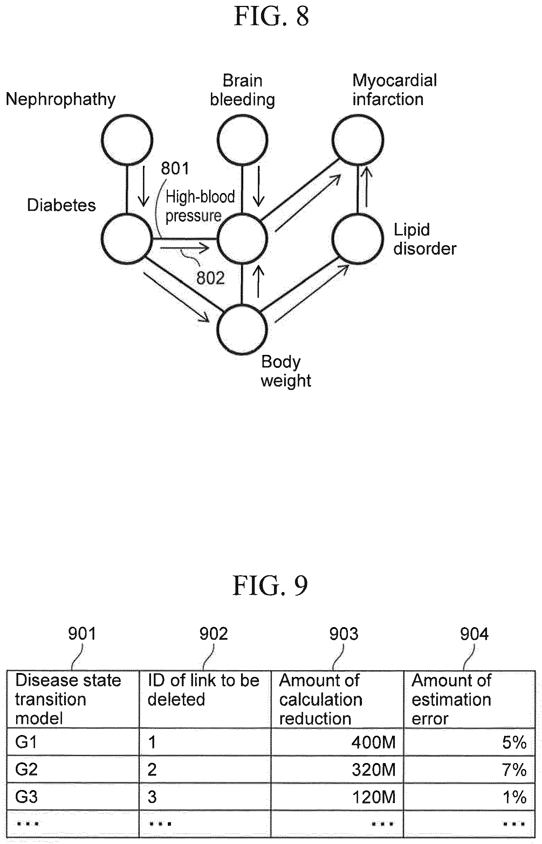

In the message-passing algorithm, as indicated by the arrows in FIG. 8, messages are passed along the links, and the probability distribution of the random variables is calculated by multiplying the received messages. The content of a message that is passed varies depending on the probability distribution of the random variable on the transmission side. In the present embodiment, the magnitude of inference error is estimated by assuming a plurality of states that could be sent via a deleted link. Specifically, with respect to a state sent via a deleted link, a plurality of states that the transmission-side random variable could take is assumed, and a message is passed. Here, two or more types of messages having the greatest difference imaginable with respect to a link deleted after model modification are passed, and the difference in their inference results is examined to estimate an error. By a similar process, it is also possible to determine an inference error of probabilistic inference techniques other than the message-passing algorithm, such as the bucket elimination algorithm, for example.

For example, in FIG. 8, when it is desired to examine the inference error of the incidence rate of myocardial infarction when link 801 is deleted, two messages are assumed for the content of a message 802 sent via link 801, i.e., a message that "100% onset of diabetes" and a message "100% no-onset of diabetes".

In a state in which the respective messages are assumed, each message is passed to the disease state transition model (model of FIG. 6) from which the link 801 is deleted, and the incidence rate of myocardial infarction is inferred. In this case, with respect to the disease state transition model from which the link 801 is deleted, two incidence rates of myocardial infarction are obtained as the result. The difference between the two incidence rates is the maximum expected error, and is considered the inference error in the incidence rate of myocardial infarction when the link 801 is deleted. When three or more states that a random variable can take could be expected, such as in the case of body weight, the inference error can be determined by passing messages assuming as many states, and performing a similar process.

The at least two messages with respect to each link that are passed when the link is deleted may be registered in the storage medium 107 in advance. For example, an identifier (link ID) identifying the link may be defined for each link, and information associating the link ID with at least two messages that are passed upon deletion of the link may be registered in the storage medium 107. By referring to the information, the inference error estimation unit 203 can determine the inference error with respect to a plurality of disease state transition models.

FIG. 9 is an example of a table showing the results of determination of the amount of calculation and inference error with respect to a plurality of modified disease state transition models. For each of the links in the pre-modification disease state transition model, a link ID is defined. The model modification execution unit 202 creates the plurality of disease state transition models G1, G2, and G3 by changing the links that are deleted, as described above. The inference calculation cost estimation unit 204 calculates the inference calculation cost of each of the modified models G1, G2, and G3. Further, the inference error estimation unit 203 calculates the inference error of each of the modified models G1, G2, and G3. Finally, based on the above information, the adopted model selection unit 205 may create information such as shown in FIG. 9. In the table of FIG. 9, there are stored, in association with each other: an identifier 901 of the disease state transition model after modification; an ID 902 of the deleted link in each disease state transition model; the amount of calculation reduction 903 with respect to each disease state transition model; and an inference error 904 with respect to a specific event in each disease state transition model. Accordingly, the modified disease state transition models G1, G2, and G3 can be compared.

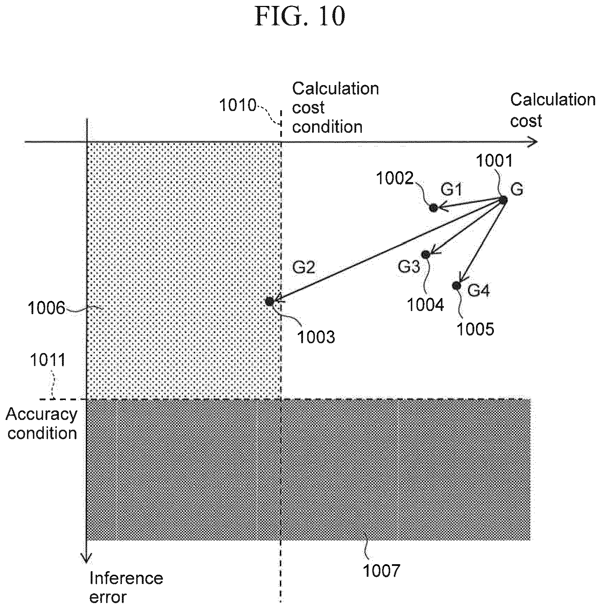

With reference to FIG. 10, the process in step 405 will be described in which the adopted model selection unit 205, on the basis of the estimated inference error and inference calculation cost of each of the disease state transition models G1, G2, G3, ..., and Gn, determines the disease state transition model Gi to be adopted. FIG. 10 describes the process of the adopted model selection unit 205. FIG. 10 is a plot of the disease state transition models on a graph of which the horizontal axis shows calculation cost and the vertical axis shows inference error.

The adopted model selection unit 205 selects, from among the plurality of disease state transition models G1, G2, G3, ..., and Gn, a disease state transition model Gi of which the ratio of the amount of decrease in calculation cost relative to the amount of increase in inference error is large. In FIG. 10, the model prior to modification is G (1001). The models after modification are G1 (1002), G2 (1003), G3 (1004), and G4 (1005). With respect to the model G (1001) prior to modification, the model of which the ratio of the amount of decrease in calculation cost relative to the amount of increase in inference error is large is G1 (1002), in light of the inclination of the arrow. Accordingly, the adopted model selection unit 205 selects G1 (1002) as Gi.

However, if any of the modified models satisfies the entered probabilistic inference conditions, that model may be selected as Gi. In FIG. 10, a broken line 1010 is a threshold value indicating the calculation cost condition entered in FIG. 11, and a broken line 1011 is a threshold value indicating the inference error condition entered in FIG. 11. Accordingly, a region 1006 is a region representing the entered probabilistic inference conditions. In this case, G2 (1003) is in the region 1006 and therefore satisfies the probabilistic inference conditions, so that G2 (1003) may be selected as Gi. When a link in a Bayesian network is deleted, the calculation cost decreases without fail and the inference error is in many cases increased. Accordingly, the above-described method can be said to be a highly effective modification method.

With reference to FIG. 10, the process in step 406, which is an end determination process, will be described. The adopted model selection unit 205 determines whether the modified model Gi is in the region 1006. If the modified model Gi is in the region 1006, the modified model Gi already satisfies the probabilistic inference conditions, so that a determination for ending the model modification process is made. Then, the post-modification model output unit 206 outputs the modified model Gi as the adopted model (step 408).

In FIG. 10, a region 1007 is a region in which the inference error condition is not satisfied, or both of the inference error and calculation cost conditions are not satisfied. If Gi is in the region 1007, it can be said that Gi will not enter the region 1006 satisfying the probabilistic inference conditions even if the model modification process is continued. This is because, when a link in a Bayesian network is deleted, the calculation cost is increased without fail and the inference error is in many cases increased. Accordingly, when Gi is in the region 1007, for example, it is determined that there is no possibility of the probabilistic inference conditions being satisfied by continuing the process, and a determination to end the process is made (namely, the process proceeds to step 408).

If Gi is not in the region 1006 nor 1007, i.e., when the modified model Gi does not satisfy the probabilistic inference conditions, and when there is the possibility of the probabilistic inference conditions being satisfied by continuing the process of the model modification execution unit 202, it is determined to continue the modification process by the model modification execution unit 202 using the modified model Gi (namely, the process proceeds to step 407). In this way, the process of steps 402 to 407 is repeatedly executed until the probabilistic inference conditions are satisfied.

Examples of inputs and outputs in the disease onset prediction device according to the present embodiment will be described. Table 1 illustrates an example in which the present embodiment is applied for future disease onset prediction and medical expenses prediction. As illustrated in Table 1, the output content may include not only probability such as the onset probability of various diseases, but also expected values of medical expenses for the next year, for example.

Table 1

| Input | Current measurement values: <br>    Age, body weight, height, blood pressure, neutral fat, etc. <br><br> Lifestyle habits: <br>    Presence/absence of exercise, walking speed, pace of eating, time for supper, sleep time, etc. <br><br> Clinical history: <br>    Medical acts received, etc. (medical records) <br>    Presence/absence of diabetes, presence/absence of high-blood pressure, presence/absence of lipid disorder, etc. |
|---|---|
| Output | Disease-by-disease onset probability for next year: <br>    Diabetes A%, high-blood pressure B%, brain bleeding C%, myocardial infarction D%, nephropathy E%, etc. <br><br> Expected value of medical expenses for next year: <br>    XX yen |

Table 2 illustrates an example of application of the present embodiment for future measurement value prediction based on lifestyle habits. The predicted values of the measurement values, such as body weight and blood pressure, as output results are not limited to specific numerical values. A measurement value range may be divided into a plurality of levels, and information of a level corresponding to a measurement value may be output.

Table 2

| Input | Current measurement values: <br>    Age, body weight, height, blood pressure, neutral fat, etc. <br><br> Lifestyle habits: <br>    Presence/absence of exercise, walking speed, pace of eating, time for supper, sleep time, etc. |
|---|---|
| Output | Predicted values of future measurement values: <br>    Body weight X kg, blood pressure Y mmHg, neutral fat Z mg/dl, etc. |

Table 3 illustrates an example of application of the present embodiment for lifestyle habits estimation.

Table 3

| Input | Current measurement values: <br>    Age, body weight, height, blood pressure, neutral fat, etc. <br><br> Clinical history: <br>    Medical acts received, etc.(medical records) <br>    Presence/absence of diabetes, presence/absence of high-blood pressure, presence/absence of lipid disorder, etc. |
|---|---|
| Output | Lifestyle habits: <br>    Presence/absence of exercise, walking speed (fast/slow), pace of eating (early/late) <br> Time for supper (early/late), sleep time (long/short), etc. |

The output content is also not limited to the information about prediction/estimation by probabilistic inference. Information about the adopted modified model Gi and a maximum amount of error (such as the inference error information in FIG. 9) that could be caused in the estimated value of a specific event in the model Gi may also be displayed on the output unit 109.

As described above, according to the disease onset prediction device of the present embodiment, when known medical data about the subject of analysis are input, and the future onset probability of a specific disease is estimated by probabilistic inference performed on a disease state transition model which is a Bayesian network, an estimation result can be output accurately within the entered probabilistic inference conditions and at small calculation cost.

In addition, compared with a conventional similar technique as according to Patent Literature 1, accuracy evaluation of a modified probabilistic inference model can be performed at high speed, whereby a probabilistic inference model which has low calculation cost and which is highly accurate can be discovered from among a number of candidates. Further, a maximum amount of error that could be caused in the estimated value of a specific event can be presented prior to the execution of inference.

Further, the present embodiment provides an approximate inference technique which enables probabilistic inference for a designated specific event at high speed and with high accuracy, and which, when adjusting the balance between the accuracy of probabilistic inference and the amount of calculation, enables adjustment focusing on the inference accuracy of a specific event.

Second embodiment

According to the present embodiment, the model modification execution unit 202 will be described which, in the first embodiment disease onset prediction device, is enabled to output a disease state transition model that enables highly accurate and high-speed probabilistic inference when the mutual information amounts of the random variables in the disease state transition model are given, or when the mutual information amounts of the random variables can be calculated from the disease state transition model.

The process in step 402 of the model modification execution unit 202 according to the present embodiment will be described with reference to FIG. 12. In step 402 of the present embodiment, first, the model modification execution unit 202 performs clustering of random variables using the mutual information amounts of the random variables as a distance. Then, the model modification execution unit 202 deletes a link connecting the clusters, on the basis of the clustering result. The clustering may be performed by an algorithm, such as k-means clustering. Thereafter, clusters other than those that include the random variables designated by probabilistic inference conditions are deleted.

For example, FIG. 12 illustrates the case where, by the clustering by the model modification execution unit 202, a cluster 1201, a cluster 1202, and a cluster 1203 have been created. Here, it is supposed that random variables designated by probabilistic inference conditions are included in the cluster 1201. In this case, links 1204, 1205, 1206, and 1207 are deleted. In addition, because the random variables designated by the probabilistic inference conditions are included in the cluster 1201, the clusters 1202 and 1203 are deleted. That is, the model modification execution unit 202 creates, as a model after modification, a model configured only of the cluster 1201 including the random variables designated by the probabilistic inference conditions.

Through the above-described process, the model modification execution unit 202 creates a disease state transition model that has a high likelihood of greatly decreasing the calculation cost of the probabilistic inference process for estimating the random variables designated by the probabilistic inference conditions. It should be noted that the model modification process according to the present embodiment is not limited to the above-described process. For example, the model modification execution unit 202 may leave some clusters other than the cluster 1201 including the random variables designated by the probabilistic inference conditions. The model modification execution unit 202 may create a model by selecting a plurality of any desired clusters from all of the created clusters. The model modification execution unit 202 may also change the granularity of the created clusters as desired, and may create clusters with finer granularity.

Third embodiment

According to the present embodiment, the process of the inference error estimation unit 203 in step 404 for calculating the estimated inference error of each of the disease state transition models G1, G2, G3, ..., and Gn differently from the first embodiment will be described.

When the inference error of a certain specific random variable X is to be determined, a plurality of conceivable states (for example, a first state and a second state) of the specific random variable X is assumed. Then, the maximum likelihood value of random variables other than the specific random variable X when the probabilistic inference process is performed on the assumption of the first state is determined. In a state where the maximum likelihood value in the first state is set, a first difference in the occurrence probability of the specific random variable X when the probabilistic inference process is performed using the modified probabilistic inference model and the pre-modification probabilistic inference model is determined. Then, the maximum likelihood value of the random variables other than the specific random variable X when the probabilistic inference process is performed on the assumption of the second state is determined. In a state where the maximum likelihood value in the second state is set, a second difference in the occurrence probability of the specific random variable X when the probabilistic inference process is performed using the modified probabilistic inference model and the pre-modification probabilistic inference model is determined. Then, the maximum of the first difference and the second difference is output as the magnitude of inference error.

The above content will be described with reference to FIG. 5 and FIG. 6. Considered is an example of determining the inference error in the inference result for the incidence rate of myocardial infarction when the probabilistic inference is performed using the Bayesian network of FIG. 6, compared with when the probabilistic inference is performed using the Bayesian network of FIG. 5. Initially, a state of "100% onset of myocardial infarction" is assumed. Under this condition, when probabilistic inference is performed using the Bayesian network of FIG. 5, the maximum likelihood values of the other random variables (nephropathy, diabetes, high-blood pressure, etc.) are determined. A set of those maximum likelihood values is S1. The maximum likelihood values herein refer to the state of the random variables with the highest occurrence probability. Then, under the condition in which S1 is assumed, probabilistic inference is performed using the Bayesian network of FIG. 5 and the Bayesian network of FIG. 6, and the difference between two incidence rates of myocardial infarction that are output as the result is determined. This difference is E1.

Then, the above-described process is performed on the assumption of the state of "100% no-onset of myocardial infarction", and a difference E2 is obtained between the two incidence rates of myocardial infarction when probabilistic inference is performed using the Bayesian network of FIG. 5 and the Bayesian network of FIG. 6. Finally, the inference error estimation unit 203 outputs the maximum of E1 and E2 as the inference error regarding the incidence rate of myocardial infarction in the Bayesian network of FIG. 6.

In the foregoing, the inference error of random variable that could take the two states of "onset of myocardial infarction" and "no onset of myocardial infarction" are determined. However, when the number of possible states of the random variable is N, N states, i.e., "100% first state", "100% second state", "100% third state",..., may be assumed. By the above process, the inference error estimation unit 203 may determine the estimated inference errors for the disease state transition models G1, G2, G3, ..., and Gn.

With the inference error estimation process according to the third embodiment, even when a plurality of links is deleted at once, error estimation can be performed by performing probabilistic inference N times. On the other hand, in the case of the inference error estimation process according to the first embodiment, it is necessary to perform probabilistic inference assuming N states for each of the deleted links, so that, as a result, the number of times of probabilistic inference required becomes large when a plurality of links is deleted. Thus, the method according to the first embodiment or the method according to the third embodiment may be selectively used as needed in accordance with the number of the links to be deleted.

The present invention is not limited to the foregoing embodiments and may include various modifications. The embodiments have been described for the purpose of facilitating an understanding of the present invention, and are not necessarily limited to be provided with all of the elements described. Some of the elements of one embodiment may be substituted with elements of another embodiment, or, alternatively, elements of the other embodiment may be incorporated into the elements of the one embodiment. With respect to some of the elements of each embodiment, addition, deletion, and/or substation of other elements may be made.

The functions, processes, means and the like of the disease onset prediction device may be implemented by means of software when a program for implementing the functions is interpreted and executed by a processor. Information about programs, tables, files and the like for implementing the functions may be placed in a storage device such as a memory, a hard disk, or a solid state drive (SSD), or in a storage medium such as an IC card, an SD card, or a DVD. The functions, processes, means and the like of the above-described disease onset prediction device may be partly or entirely designed in the form of an integrated circuit for hardware implementation.

Reference Signs List

| | |
|---|---|
| 101 | Disease state transition model input unit |
| 102 | Disease state transition model modification unit |
| 103 | Probabilistic inference condition input unit |
| 104 | Analysis subject medical data input unit |
| 105 | Probabilistic inference execution unit |
| 106 | Prediction result output unit |
| 107 | Storage medium |
| 108 | Input unit |
| 109 | Output unit |
| 110 | Computing device |
| 111 | Memory |
| 201 | Pre-modification model input unit |

202    Model modification execution unit
203    Inference error estimation unit
204    Inference calculation cost estimation unit
205    Adopted model selection unit
206    Post-modification model output unit

**Claims**

1.  A probabilistic inference system comprising:

    a pre-modification model input unit that receives an input of a probabilistic inference model;
    a model modification execution unit that outputs a modified probabilistic inference model by modifying the probabilistic inference model;
    an inference calculation cost estimation unit that calculates a calculation cost when a probabilistic inference process is performed using the modified probabilistic inference model;
    an inference error estimation unit that estimates a magnitude of inference error that can be caused in a certain designated random variable in the probabilistic inference model when the probabilistic inference process is performed using the modified probabilistic inference model, compared with when the probabilistic inference process is performed using the probabilistic inference model;
    an adopted model selection unit that selects a probabilistic inference model to be adopted based on a probabilistic inference condition regarding the calculation cost and the inference error; and
    a post-modification model output unit that outputs the adopted probabilistic inference model.

2.  The probabilistic inference system according to claim 1, wherein:

    the probabilistic inference model is a graphical model including random variables and a link representing probabilistic dependency between the random variables; and
    the model modification execution unit creates the modified probabilistic inference model by deleting the link.

3.  The probabilistic inference system according to claim 2, wherein the inference error estimation unit estimates the magnitude of inference error by assuming a plurality of states that could be sent via the deleted link.

4.  The probabilistic inference system according to claim 3, wherein the plurality of states are states with a maximum conceivable difference with respect to the deleted link.

5.  The probabilistic inference system according to claim 1, wherein the adopted model selection unit selects, from modified probabilistic inference models, one with the largest ratio of an amount of decrease in the calculation cost to an amount of increase in the inference error, and determines whether the selected model satisfies the probabilistic inference condition.

6.  The probabilistic inference system according to claim 5, wherein:

    when the selected model satisfies the probabilistic inference condition, the post-modification model output unit outputs the selected model as the adopted probabilistic inference model; and
    when the selected model does not satisfy the probabilistic inference condition, and when there is a possibility of the probabilistic inference condition being satisfied by continuing the process of the model modification execution unit, the modification process by the model modification execution unit is continued using the selected model.

7.  The probabilistic inference system according to claim 6, wherein the modification process by the model modification execution unit is repeatedly executed until the probabilistic inference condition is satisfied.

8.  The probabilistic inference system according to claim 1, wherein the model modification execution unit performs clustering of random variables in the probabilistic inference model, and creates the modified probabilistic inference model by selecting any desired cluster from a plurality of created clusters.

9.  The probabilistic inference system according to claim 8, wherein the model modification execution unit creates the modified probabilistic inference model configured only of clusters including random variables designated by the probabilistic inference condition.

10. The probabilistic inference system according to claim 1, wherein the inference error estimation unit, when determining the inference error of a certain specific random variable,

determines a maximum likelihood value when the probabilistic inference process is performed assuming each of a plurality of conceivable states of the specific random variable, with respect to random variables other than the specific random variable, and

calculates a difference in the occurrence probability of the specific random variable when the probabilistic inference process is performed using the modified probabilistic inference model and the probabilistic inference model prior to modification, in a state in which the maximum likelihood value is set.

11. The probabilistic inference system according to claim 10, wherein the inference error estimation unit outputs, as the magnitude of inference error, a maximum difference of

a difference in the occurrence probability of the specific random variable when, in a state in which the maximum likelihood value in a first state among the plurality of states is set, the probabilistic inference process is performed using the modified probabilistic inference model and the probabilistic inference model prior to modification, and

a difference in the occurrence probability of the specific random variable when, in a state in which the maximum likelihood value in a second state among the plurality of states is set, the probabilistic inference process is performed using the modified probabilistic inference model and the probabilistic inference model prior to modification.

12. The probabilistic inference system according to claim 1, wherein the probabilistic inference model is a Bayesian network.

13. The probabilistic inference system according to claim 12, wherein the probabilistic inference process is probabilistic inference using an algorithm including a message-passing algorithm.

14. The probabilistic inference system according to claim 12, wherein the probabilistic inference process is probabilistic inference using an algorithm including a bucket elimination algorithm.

15. The probabilistic inference system according to claim 1, further comprising:

a probabilistic inference condition input unit that accepts an input of the probabilistic inference condition;
a data input unit that accepts input data to the probabilistic inference model;
a probabilistic inference execution unit that executes the probabilistic inference process using the adopted probabilistic inference model; and
a prediction result output unit that outputs a result from the probabilistic inference execution unit.

# FIG. 1

Disease onset prediction device

Storage medium — 107

Input unit — 108

Output unit — 109

Computing device — 110

Memory — 111

Disease state transition model input unit — 101

Disease state transition model modification unit — 102

Probabilistic inference condition input unit — 103

Analysis subject medical data input unit — 104

Probabilistic inference execution unit — 105

Prediction result output unit — 106

# FIG. 2

Disease state transition model
modification unit

| |
|---|
| Pre-modification model input unit — 201 |
| Model modification execution unit — 202 |
| Inference error estimation unit — 203 |
| Inference calculation cost estimation unit — 204 |
| Adopted model selection unit — 205 |
| Post-modification model output unit — 206 |

# FIG. 3

Start of disease onset
prediction process

Receive input of disease state
transition model —— 301

Receive input of probabilistic
inference conditions —— 302

Modify disease state transition
model based on probabilistic
inference conditions —— 303

Receive input of data for
inference —— 304

Perform probabilistic inference
with respect to data for
inference, using modified model —— 305

306

Other data for inference? —— NO

YES

Output results —— 307

End of disease onset
prediction process

# FIG. 4

```
┌─────────────────────────────┐
│   Start of model modification│
│            process          │
└─────────────────────────────┘
              │
              ▼
┌─────────────────────────────┐
│ Receive disease state       │── 401
│ transition model G          │
└─────────────────────────────┘
              │
              ▼
┌─────────────────────────────┐
│ Modify G, and create G1, G2,│── 402
│ G3, ...                     │
└─────────────────────────────┘
              │
              ▼
┌─────────────────────────────┐
│ Evaluate inference          │── 403
│ calculation cost            │
│ for G1, G2, G3, ...         │
└─────────────────────────────┘
              │
              ▼
┌─────────────────────────────┐
│ Evaluate inference accuracy │── 404
│ of G1, G2, G3, ...          │
└─────────────────────────────┘
              │
              ▼
┌─────────────────────────────┐
│ Based on inference accuracy │── 405
│ and inference calculation   │
│ cost for G1, G2, G3, ...,   │
│ determine Gi to be adopted  │
│ from G1, G2, G3, ...        │
└─────────────────────────────┘
              │
              ▼
         ╱───────────╲
        ╱   Based     ╲ ── 406
       ╱ on inference   ╲
      ╱ accuracy and     ╲ ──── YES
      ╲ inference calc.   ╱
       ╲ cost for Gi,    ╱
        ╲ end model     ╱
         ╲ modification ╱
          ╲ process?   ╱
              │NO              │
              ▼                ▼
┌─────────────────────┐  ┌─────────────────────────┐
│ Set Gi as G         │  │ Output disease          │── 408
│ from subsequent     │  │ transition model Gi     │
│ process  ── 407     │  └─────────────────────────┘
└─────────────────────┘              │
                                     ▼
                          ┌─────────────────────────┐
                          │ End of model            │
                          │ modification process    │
                          └─────────────────────────┘
```

FIG. 5

Nephrophathy

Brain
bleeding

Myocardial
infarction

High-blood
pressure

Diabetes

Lipid
disorder

Body
weight

FIG. 6

Nephrophathy

Brain
bleeding

Myocardial
infarction

High-blood
pressure

Diabetes

Lipid
disorder

Body
weight

EP 3 125 161 A1

FIG. 7

705    706

701    702    703    704

# FIG. 8

# FIG. 9

| Disease state transition model | ID of link to be deleted | Amount of calculation reduction | Amount of estimation error |
|---|---|---|---|
| G1 | 1 | 400M | 5% |
| G2 | 2 | 320M | 7% |
| G3 | 3 | 120M | 1% |
| ... | ... | ... | ... |

901   902   903   904

# FIG. 10

# FIG. 11

| Inference accuracy | Inference error of | Diabetes | be | 1 | % or less |

Inference accuracy | Inference error of Diabetes be 1 % or less

1101  1102  1100

Inference speed  2000  msec or less

1103

Enter  1104

# FIG. 12

1207

1205

1201

1202

1204

1206

1203

## INTERNATIONAL SEARCH REPORT

| International application No. |
|---|
| PCT/JP2014/058166 |

| A. | CLASSIFICATION OF SUBJECT MATTER |
|---|---|

*G06N5/04*(2006.01)i, *G06Q50/22*(2012.01)i

According to International Patent Classification (IPC) or to both national classification and IPC

| B. | FIELDS SEARCHED |
|---|---|

Minimum documentation searched (classification system followed by classification symbols)
G06N5/04, G06N7/00, G06Q50/22

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)
JSTPlus(JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2007-66260 A  (NTT Docomo Inc.), 15 March 2007 (15.03.2007), paragraphs [0014] to [0021] (Family: none) | 1-15 |
| A | US 8447710 B1  (Sreerupa DAS), 21 May 2013 (21.05.2013), abstract; column 1, lines 31 to 39; column 5, line 22 to column 7, line 53 (Family: none) | 1-15 |
| A | Naoya MURAO et al., "Empirical Investigations on the parallelized Bayesian Optimization Algorithm", IPSJ SIG Notes, 03 March 2004 (03.03.2004), vol.2004, no.20, pages 169 to 174, particularly, passage 2 | 1-15 |

☐  Further documents are listed in the continuation of Box C.     ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered  to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 June, 2014 (03.06.14) | 17 June, 2014 (17.06.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**EP 3 125 161 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8447710 B